# EUROPÄISCHE PATENTSCHRIFT

(11) **EP 1 341 754 B1**
(45) Veröffentlichungstag und Bekanntmachung des Hinweises auf die Patenterteilung: **08.12.2004**
(21) Anmeldenummer: 01994713.4
(22) Anmeldetag: 24.11.2001
(51) Int. Cl.: C07C 311/39, C07D 213/42, C07D 307/52, C07D 333/20, A61K 31/18, A61P 9/10, A61P 25/28, A61P 35/00

(54) **SUBSTITUIERTE ANTHRANILSÄUREN**
SUBSTITUTED ANTHRANILIC ACIDS
ACIDES ANTHRANILIQUES SUBSTITUES

(30) Priorität: 07.12.2000 DE 10060809
(43) Veröffentlichungstag der Anmeldung: 10.09.2003
(73) Patentinhaber: Aventis Pharma Deutschland GmbH, 65929 Frankfurt am Main (DE)
(72) Erfinder: WEICHERT, Andreas, 22846 Norderstedt (DE); JANSEN, Hans-Willi, 65527 Niedernhausen (DE); KLEEMANN, Heinz-Werner, 65474 Bischofsheim (DE); LANG, Hans-Jochen, 65719 Hofheim (DE); RÜTTEN, Hartmut, 65510 Idstein (DE)
(86) Internationale Anmeldenummer: PCT/EP2001/013681
(87) Internationale Veröffentlichungsnummer: WO 2002/046148

(56) Entgegenhaltungen:
- EP-A- 0 604 852
- EP-A- 0 726 250
- DE-A- 1 802 208
- US-A- 3 565 920

## Beschreibung

Die Erfindung betrifft Anthranilsäuren der Formel I 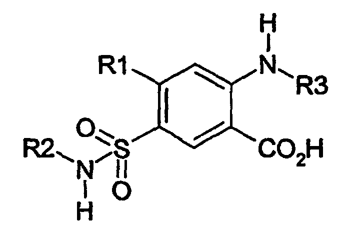 worin bedeuten:
- R(1): H, Cl, Br, I, CN, (C₁-C₈)- Alkyl, (C₃-C₆)- Cycloalkyl oder Phenyl,
wobei der aromatische Kern unsubstituiert oder substituiert ist mit 1-3 Substituenten aus der Gruppe F, Cl, (C₁-C₃)- Alkyl, Methoxy oder -(CF₂)ₐ-CF₃;
a Null, 1, 2 oder 3;
- R(2): (C₁-C₈)- Alkyl, -C_{b}H_{2b}- (C₃-C₆)- Cycloalkyl, -C_{b}H_{2b}- Phenyl, -C_{b}H_{2b}-Pyridinyl, -C_{b}H_{2b}- Thiophenyi, -C_{b}H_{2b}- Furanyi,
wobei die aromatischen Systeme unsubstituiert oder substituiert sind mit 1-3 Substituenten aus der Gruppe F, Cl, CF₃, (C₁-C₃)-Alkyl, Methoxy oder -SO₂NR(4)R(5);
R(4) und R(5) unabhängig voneinander H, (C₁-C₄)-Alkyl,
b Null, 1, 2, 3 oder 4;
- R(3): -C_{d}H_{2d}- Phenyl,
wobei der aromatische Kern unsubstituiert oder substituiert ist mit 1-3 Substituenten aus der Gruppe F, Cl, CF₃, (C₁-C₃)- Alkyl oder Methoxy;
d 3 oder 4;
sowie deren pharmazeutisch verträgliche Salze.

Bevorzugt sind Verbindungen der Formel I, in denen bedeuten:
- R(1): Cl, (C₁-C₄)- Alkyl oder Phenyl,
wobei der aromatische Kern unsubstituiert oder substituiert ist mit 1-3 Substituenten ausgewählt aus der Gruppe bestehend aus F, Cl, CF₃, (C₁-C₃)- Alkyl oder Methoxy;
- R(2): (C₁-C₄)- Alkyl, -C_{b}H_{2b}- Cyclohexyl, -C_{b}H_{2b}- Phenyl, -C_{b}H_{2b}- Pyridinyl, -C_{b}H_{2b}- Thiophenyl, -C_{b}H_{2b}- Furanyl,
wobei die aromatischen Systeme unsubstituiert oder substituiert sind mit 1 - 3 Substituenten ausgewählt aus der Gruppe bestehend aus F, Cl, CF₃, (C₁-C₃)-Alkyl, Methoxy oder -SO₂NH₂;
b Null, 1 oder 2;
- R(3): -n-C₄H₈- Phenyl,
wobei das Phenyl unsubstituiert ist oder substituiert mit 1 - 3 Substituenten ausgewählt aus der Gruppe bestehend aus F, Cl, CF₃, (C₁-C₃)- Alkyl oder Methoxy;
sowie deren pharmazeutische verträgliche Salze.

Besonders bevorzugt sind Verbindungen der Formel I, in denen bedeuten:
- R(1): Cl, (C₁-C₄)- Alkyl oder Phenyl,
wobei der aromatische Kern unsubstituiert oder substituiert ist mit 1-3 Substituenten ausgewählt aus der Gruppe bestehend aus F, Cl, CF₃, (C₁-C₃)-Alkyl oder Methoxy;
- R(2): (C₁-C₄)- Alkyl, -C_{b}H_{2b}- Cyclohexyl, -C_{b}H_{2b}- Phenyl, -C_{b}H_{2b}- Pyridinyl, - C_{b}H_{2b}- Thiophenyl, -C_{b}H_{2b}- Furanyl,
wobei die aromatischen Systeme unsubstituiert oder substituiert sind mit 1 - 3 Substituenten ausgewählt aus der Gruppe bestehend aus F, Cl, CF₃, (C₁-C₃)- Alkyl, Methoxy oder -SO₂NH₂;
b 1;
- R(3): -n-C₄H₈- Phenyl,
sowie deren pharmazeutisch verträgliche Salze.

Ganz besonders bevorzugt ist die Verbindung 4- Chtoro-5-(3-chloro-4-fluoro-benzylsulfamoyl)-2-phenylbutylamino-benzoesäure, sowie ihre pharmazeutisch verträglichen Salze.

Enthält einer der Substituenten R(1) bis R(5) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen. Verbindungen der Formel I lassen sich durch den Fachmann nach aus der Literatur bekannten Verfahren synthetisieren.

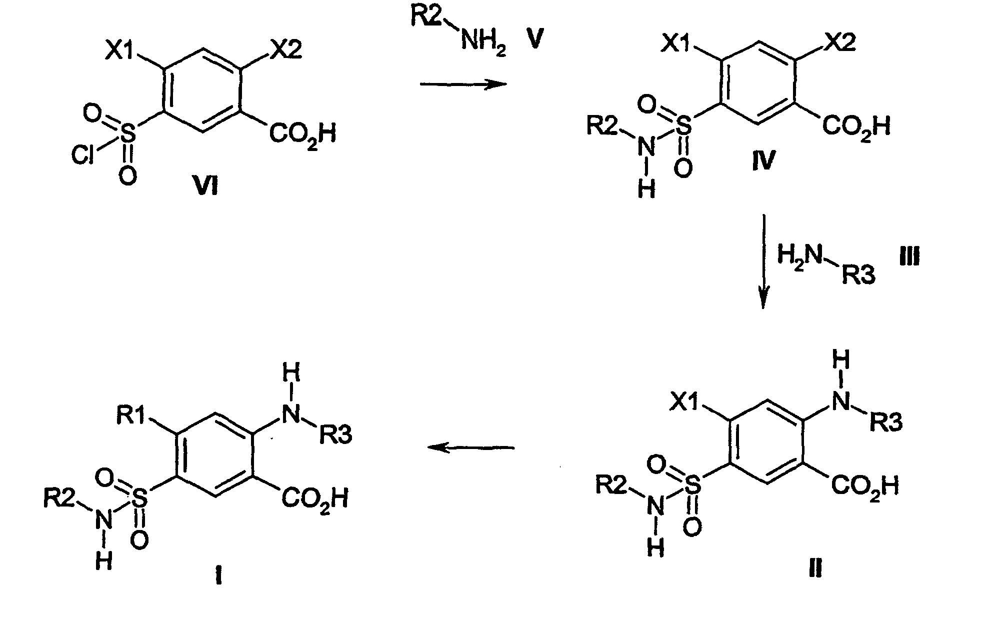

Als mögliche Fluchtgruppen X2 (Formel VI) in der nucleophilen aromatischen Substitutionsreaktion mit Aminen III können Fluor oder Chlor in Betracht gezogen werden.
Die Einführung einiger Substituenten in 4-Stellung von Zwischenprodukt II (X1 gleich Brom, lod oder -O-SO₂CF₃ gelingt durch ebenfalls literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Anthranilsäuren I sind im allgemeinen schwache Säuren, die Basen unter Bildung von Salzen binden. Als Baseadditionsprodukte kommen alle pharmakologisch verträglichen Salze infrage, beispielsweise Alkalisalze, Lysinate und Tris-(hydroxymethyl)-methylamin-Salze.

Die Verbindungen I sind substituierte Anthranilsäuren.

Ein prominenter Vertreter der Anthranilsäureklasse ist das Furfurylderivat Furosemid, das als Diuretikum in der Therapie Verwendung findet. Furosemid inhibiert den Natrium/Kalium/2Chlor-Cotransporter im aufsteigenden Ast der Henleschen-Schleife in der Niere. 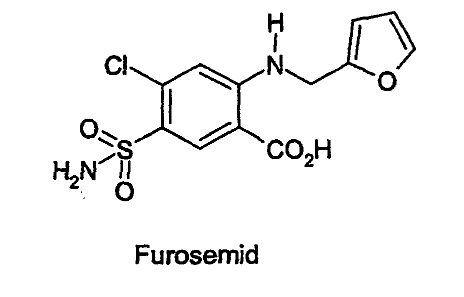 In der DE 18 02 208 werden strukturell ähnliche Anthranilsäuren beschrieben, die jedoch an R3 ausschließlich Benzyl, Furfuryl- oder Thienylsubstituenten tragen und diuretische Wirkung besitzen. Im US-Patent 3 565 920 werden Anthranilsäuren beansprucht, die strukturell mit den Verbindungen der Formel I verwandt sind, sich aber ebenfalls durch eine starke salidiuretische Wirksamkeit auszeichnen.

Es war daher überraschend, dass die erfindungsgemäßen Verbindungen keine unerwünschten und nachteiligen salidiuretischen, jedoch sehr gute cardioprotektive Eigenschaften aufweisen, beispielsweise bei Sauerstoffmangelerscheinungen. Die Verbindungen sind infolge ihrer pharmakologischen Eigenschaften als cardioprotektive Arzneimittel zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/HCO₃⁻ Cotransporters (NBC) als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z. B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Darüber hinaus reduzieren die erfindungsgemäßen Verbindungen die Entstehung bzw. das Ausmaß einer Herzinsuffizienz nach verschiedenen Insulten. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Himödems geeignet sind. Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden. Die Erfindung betrifft weiterhin eine Kombination eines NBC-Blockers der Formel I mit Natrium/Wasserstoff-Austausch (NHE)-Inhibitoren. Beide Wirkstoffklassen zeigen in der kombinierten therapeutischen Anwendung überraschenderweise synergistische Effekte bei der Behandlung von Krankheitsbildern, die auf ischämische Zustände und Reperfusionsereignisse zurückzuführen sind. Somit sind die Kombinationen eines NBC-Inhibitors mit einem NHE-Blocker hervorragend zur Infarkt- und Reinfarktprophylaxe und der Infarktbehandlung sowie zur Behandlung der angina pectoris und der Inhibierung ischämisch induzierter Herzarrhythmien, der Tachykardie und der Entstehung und Aufrechterhaltung des Kammerflimmerns geeignet, wobei die Kombinationen auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen infolge verstärkter Inhibierung des Na⁺ Einstroms in die Zelle als Arzneimittel zur Behandlung aller akuten oder chronischen, durch Ischämie ausgelösten Schäden oder dadurch primär oder sekundär induzierter Krankheiten verwendet werden. Dies betrifft ihre Verwendung als Arzneimittel für operative Eingriffe, z. B. bei Organtransplantationen, wobei die Kombinationen eines NHE-Inhibitors mit einem Blocker des nicht-inaktivierenden Natriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NBC-Blockers der Formel I mit NHE-Inhibitoren sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind diese Kombinationen auch als Arzneimittel zur Behandlung von Ischämien des Nervensystems, insbesondere des Zentralnervensystems geeignet, wobei sie zur Behandlung des Schlaganfalls oder des Himödems geeignet sind. Darüber hinaus eignen sich die erfindungsgemäßen Kombinationen ebenfalls zur Behandlung von Formen des Schocks, wie beispielsweise des allergischen, cardiogenen, hypovolämischen und des bakteriellen Schocks.

So wurde beispielsweise überraschend gefunden, dass die Kombination des NBC-Blockers 4-Chloro-5-(3-chloro-4-fluoro-benzylsulfamoyl)-2-phenylbutylamino-benzoesäure mit dem NHE-Inhibitor Cariporide mesilat, 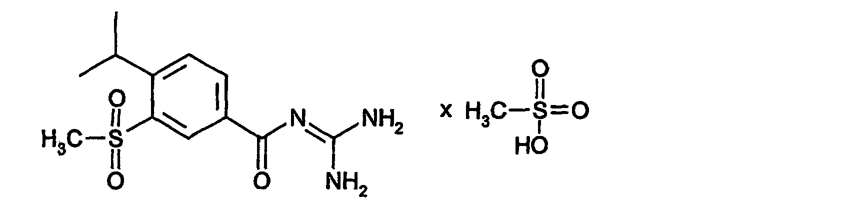 das zum Beispiel in der US-Patentschrift US 5 591 754 beschrieben ist, eine größere als additive, cardioprotektive Wirkung in einem Ischämie/Reperfusions-Modell (isoliert arbeitendes Rattenherz) zeigte.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Liste der Abkürzungen:

- ACN: Acetonitril
- HPLC: High Pressure Liqiud Chromatography
- LM: Lösungsmittel
- RT: Raumtemperatur
- EE: Ethylacetat (EtOAc)
- Smp: Schmelzpunkt
- eq.: Äquivalent
- TFA: Trifluoressigsäure
- Rt: Retentionszeit
- MS: Massenspektrometer
- dppf: 1,1'-Bis-(diphenylphosphino)-ferrocen

### Analytik:

### HPLC 1Agilent 1100

Method Gradient: 10 % ACN(0.1% TFA) / 90% water (0.1% TFA) - auf -90 % ACN(0.1% TFA)/ 10%water(0.1% TFA) in 7 min, Fluß 2.5 mL/min Säule: Altech RP18, 3 µm, 7 x 35 mm
MS: Waters Mass Lynx- ESI-TOF, [M-H⁺]⁻, 100% Peak, wenn nicht anders angegeben
HPLC 2Agilent 1100 LC/MSD
Method Gradient: 5 % ACN(0.05% TFA) / 95% water (0.05% TFA) - auf
-95 % ACN (0.05% TFA)/5%water (0.05% TFA) in 4 min, Fluß 0.5 mL/min
Säule: ESI, [M+H⁺]⁺, Merck Purospher RP18, 5 µm, 2 x 55 mm

Allgemeine Vorschrift zur Herstellung von Anthranilsäuren (I/II)

Stufe 1: 1.0 eq. der 2,4-Bis-halo-5-chlorsulfonyl-benzoesäure der Formel VI löst bzw. suspendiert man in Essigsäureethylester (5 ml/mmol) und versetzt sodann mit 5 eq. Amin der Formel V. Nach Rühren über 17 Stunden bei RT wird das Reaktionsgemisch mit 2N Salzsäure auf pH 1 bis 2 angesäuert und mit EE extrahiert. Nach Trocknen über MgSO₄ wird das LM evaporiert und das Rohprodukt ohne weitere Reinigung in die nächste Stufe eingesetzt.

Stufe 2: 1.0 eq. des 2,4-Bis-halo-5-sulfamoyl-benzoesäure-Derivats der Formel IV wird mit 5 eq. Amin der Formel III versetzt und bei 100°C über 24 Stunden erhitzt. Nach Abkühlen des Reaktionsgemisches wird mit 5N Zitronensäurelösung versetzt und mit EE extrahiert. Die organische Phase wird konzentriert und das erhaltene Rohprodukt über präparative HPLC (RP-Gel, Laufmittel Acetonitril/Wasser-Gradient) gereinigt.

### Beispiel 1: 4-Chloro-5-(3-chloro-4-fluoro-benzylsulfamoyl)-2-phenylbutylaminobenzoesäure,

Lysin-Salz, HPLC2: Rt = 5.252 MS: 525.20

### Syntheseweg:

a) 4-Chloro-5-(3-chloro-4-fluoro-benzylsulfamoyl)-2-chloro-benzoesäure aus Reaktion von 4-Chloro-5-(chlorsulfonyl)-2-chloro-benzoesäure und 4-Fluor-3-chlorbenzylamin nach allg. Vorschrift, Stufe 1.
b) 4-Chloro-5-(3-chloro-4-fluoro-benzylsulfamoyl)-2-phenylbutylamino-benzoesäure aus a) durch Reaktion mit Phenylbutylamin nach allg. Vorschrift, Stufe 2, farblose Kristalle.
c) Salzbildung mit 1 eq 1 b), gelöst in Acetonitril, unter Zugabe einer wäßrigen Lösung von 1 eq D,L-Lysin. Nach Gefriertrocknung verbleibt ein farbloser Feststoff.

### Beispiel 2: 4-Bromo-5-(3-chloro-4-fluoro-benzylsulfamoyl)-2-phenylbutylaminobenzoesäure,

HPLC2: Rt = 5.270 MS: 569.00

### Syntheseweg:

a) 4-Bromo-5-(chlorsulfonyl)-2-chloro-benzoesäure aus 4-Bromo-2-chlorobenzoesäure durch Reaktion in reiner Chlorsulfonsäure (10 eq) bei 95°C innerhalb von 6h. Nach Erkalten wird auf Eis gegossen und der Niederschlag abfiltriert, farbloser Feststoff.
b) 4-Bromo-5-(3-chloro-4-fluoro-benzylsulfamoyl)-2-chloro-benzoesäure aus 2a) nach allg. Vorschrift, Stufe 1.
c) 4-Bromo-5-(3-chloro-4-fluoro-benzylsulfamoyl)-2-phenylbutylamino-benzoesäure aus b) durch Reaktion mit Phenylbutylamin nach allg. Vorschrift, Stufe 2, farbloser Feststoff.

### Beispiel 3: 5-(3-chloro-4-fluoro-benzylsulfamoyl)-2-phenylbutylamino-benzoesäure,

HPLC2: Rt = 5.227 MS: 491.40

### Syntheseweg:

a) 5-(3-chloro-4-fluoro-benzylsulfamoyl)-2-phenylbutylamino-benzoesäure aus 2c) durch Hydrogenolyse mittels 10%-Pd/C-Katalysator in Ethanol bei RT über 2 Tage. Der Katalysator wird abfiltriert und das LM abgezogen. Die Reinigung erfolgt durch präparative RP-HPLC (Wasser/ Acetonitril-Gradient), farbloser Feststoff nach Gefriertrocknung.

### Beispiel 4: 4-Methyl-5-(3-chloro-4-fluoro-benzylsulfamoyl)-2-phenylbutylaminobenzoesäure,

HPLC2: Rt = 5.199 MS: 505.05

### Syntheseweg:

a) 4-Bromo-2-fluoro-benzoesäuremethylester aus 4-Bromo-2-fluoro-benzoesäure durch Veresterung mit einem Überschuss an Acetylchlorid in Methanol bei RT innerhalb 7h. Wässrige Aufarbeitung und anschließende Gefriertrocknung liefert einen farblosen Feststoff.
b) 4-Methyl-2-fluoro-benzoesäuremethylester aus 1 eq 4 a) durch Reaktion mit 2 eq Methylzink-chlorid, hergestellt aus dem entsprechendem Grignard-Reagenz in THF und Zinkchlorid-THF-Komplex, in Gegenwart von 5mol% Pd(dppf)Cl₂ und 6mol% Cul in THF bei RT innerhalb 18 h. Nach NH₄Cl-Aufarbeitung wird mit EE extrahiert, das LM evaporiert, das Rohprodukt über präp. HPLC gereinigt und lyophilisiert, farbloser Feststoff.
c) 4-Methyl-2-fluoro-benzoesäure aus 4 b) durch Hydrolyse mit 2N Natronlauge in Methanol bei 50°C innerhalb einer Stunde. Anschließendes Azidifizieren mit 2N Salzsäure, Extraktion mit EE und Trocknung über MgSO₄ ergibt ein Rohprodukt, welches in die nächste Stufe eingesetzt wird.
d) 4-Methyl-5-(chlorsulfonyl)-2-fluoro-benzoesäure aus 4 c) durch Reaktion in reiner Chlorsulfonsäure (10 eq) bei 95°C innerhalb von 6h. Nach Erkalten wird auf Eis gegossen, mit EE extrahiert und über MgSO₄ getrocknet. Evaporation ergibt ein gelbliches Öl, welches in dieser Reinheit weiter umgesetzt wird.
e) 4-Methyl-5-(3-chloro-4-fluoro-benzylsulfamoyl)-2-fluoro-benzoesäure aus 4 d) und 4-Fluor-3-chlor-benzylamin analog 1 a), farbloser Feststoff.
f) 4-Methyl-5-(3-chloro-4-fluoro-benzylsulfamoyl)-2-phenylbutylamino-benzoesäure aus 4 e) durch Reaktion mit Phenylbutylamin nach allg. Vorschrift, Stufe 2, farbloser Feststoff.

### Beispiel 5: 4-Isopropyl-5-(3-chloro-4-fluoro-benzylsulfamoyl)-2-phenylbutylaminobenzoesäure,

HPLC2: Rt = 5.399 MS: 533.10

### Syntheseweg:

a) 4-Isopropyl-2-fluoro-benzoesäuremethylester aus 1 eq 4 a) durch Reaktion mit 2 eq Isopropylzink-chlorid, analog zur Darstellung von 4 a), farbloser Feststoff.
b) 4-Isopropyl-2-fluoro-benzoesäure aus 5 a) durch Hydrolyse mit 2N Natronlauge, analog 4 c).
c) 4-Isopropyl-5-(chlorsulfonyl)-2-fluoro-benzoesäure aus 5 b) durch Reaktion in reiner Chlorsulfonsäure analog 4 d).
d) 4-Isopropyl-5-(3-chloro-4-fluoro-benzylsulfamoyl)-2-fluoro-benzoesäure aus 5 c) und 4-Fluor-3-chlor-benzylamin analog 1 a), farbloser Feststoff.
e) 4-Isopropyl-5-(3-chloro-4-fluoro-benzylsulfamoyl)-2-phenylbutylaminobenzoesäure aus 5 d) durch Reaktion mit Phenylbutylamin nach allg. Vorschrift, Stufe 2, farbloser Feststoff.

### Beispiel 6: 4-n-Propyl-5-(3-chloro-4-fluoro-benzylsulfamoyl)-2-phenylbutylaminobenzoesäure,

HPLC2: Rt = 5.437 MS: 533.10

### Syntheseweg:

a) 4- n-Propyl-2-fluoro-benzoesäuremethylester aus 1 eq 4 a) durch Reaktion mit 2 eq n-Propylzink-chlorid, analog zur Darstellung von 4a), farbloser Feststoff.
b) 4- n-Propyl-2-fluoro-benzoesäure aus 6 a) durch Hydrolyse mit 2N Natronlauge, analog 4 c).
c) 4- n-Propyl-5-(chlorsulfonyl)-2-fluoro-benzoesäure aus 6 b) durch Reaktion in reiner Chlorsulfonsäure analog 4 d).
d) 4- n-Propyl-5-(3-chloro-4-fluoro-benzylsulfamoyl)-2-fluoro-benzoesäure aus 6 c) und 4-Fluor-3-chlor-benzylamin analog 1 a), farbloser Feststoff.
e) 4- n-Propyl-5-(3-chloro-4-fluoro-benzylsulfamoyl)-2-phenylbutylaminobenzoesäure aus 6 d) durch Reaktion mit Phenylbutylamin nach allg. Vorschrift, Stufe 2, farbloser Feststoff.

### Beispiel 7: 4-(4-Methyl-phenyl)-5-(3-chloro-4-fluoro-benzylsulfamoyl)-2-phenylbutylamino-Benzoesäure

HPLC2: Rt = 5.762 MS: 581.60
aus Reaktion von 1 eq 4-Bromo-5-(3-chloro-4-fluoro-benzylsulfamoyl)-2-phenylbutylamino-benzoesäure und 1.2 eq 4-Tolyl-boronsäure in einem LM-Gemisch Toluol/MeOH 2 : 1 in Gegenwart von 10 mol% Pd(OAc)₂, 20 mol% Triphenylphosphin und 3 eq Natriumcarbonat unter Rückfluss für 3 Stunden. Wässrige Aufarbeitung, anschließende präparative HPLC und Gefriertrocknung liefert einen farblosen Feststoff.

### Beispiel 8: 4-Chloro-2-phenylbutylamino-5-[(pyridin-4-ylmethyl)-sulfamoyl]-benzoesäure,

HPLC1: Rt = 4.417 MS: 474.1 [M+H+]+

### Syntheseweg:

a) 2,4-Dichloro-5-[(pyridin-4-ylmethyl)-sulfamoyl]-benzoesäure aus Reaktion von 2,4-Dichloro-5-(chlorsulfonyl)-benzoesäure und Pyridin-4-yl-methylamin nach allg. Vorschrift, Stufe 1.
b) 4-Chloro-2-phenylbutylamino-5-[(pyridin-4-ylmethyl)-sulfamoyl]-benzoesäure aus a) durch Reaktion mit Phenylbutylamin nach allg. Vorschrift, Stufe 2, farbloser Feststoff.

### Beispiel 9: 4-Chloro-2-phenylbutylamino-5-[(pyridin-2-ylmethyl)-sulfamoyl]-benzoesäure

HPLC1: Rt = 4.441 MS: 474.1 [M+H+]+

### Syntheseweg:

a) 2,4-Dichloro-5-[(pyridin-2-ylmethyl)-sulfamoyl]-benzoesäure aus Reaktion von 2,4-Dichloro-5-(chlorsulfonyl)-benzoesäure und Pyridin-2-yl-methylamin nach allg. Vorschrift, Stufe 1.
b) 4-Chloro-2-phenylbutylamino-5-[(pyridin-2-ylmethyl)-sulfamoyl]-benzoesäure aus a) durch Reaktion mit Phenylbutylamin nach allg. Vorschrift, Stufe 2, farbloser Feststoff.

### Beispiel 10: 4-Chloro-2-phenylbutylamino-5-(3-chloro-benzylsulfamoyl)-benzoesäure

HPLC1: Rt = 5.267 MS: 505.08

### Syntheseweg:

a) 2,4-Dichloro-5-(3-chloro-4-fluoro-benzylsulfamoyl)-benzoesäure aus Reaktion von 2,4-Dichloro-5-(chiorsulfonyl)-benzoesäure und 3-Chloro-benzylamin nach allg. Vorschrift, Stufe 1.
b) 4-Chloro-2-phenylbutylamino-5-(3-chloro-benzylsulfamoyl)-benzoesäure aus a) durch Reaktion mit Phenylbutylamin nach allg. Vorschrift, Stufe 2, farbloser Feststoff.

### Beispiel 11: 4-Chloro-2-phenylbutylamino-5-(4-sulfamoyl-benzylsulfamoyl)-benzoesäure

HPLC1: MS: 550.13

### Syntheseweg:

a) 4-Chloro-5-(4-sulfamoyl-benzylsulfamoyl)-benzoesäure aus Reaktion von 2,4-Dichloro-5-(chlorsulfonyl)-benzoesäure und 4-Sulfamoyl-benzylamin nach allg. Vorschrift, Stufe 1.
b) 4-Chloro-2-phenylbutylamino-5-(4-sulfamoyl-benzylsulfamoyl)-benzoesäure aus a) durch Reaktion mit Phenylbutylamin nach allg. Vorschrift, Stufe 2, farbloser Feststoff.

### Beispiel 12: 4-Chloro-2-phenylbutylamino-5-(2,3-dichloro-benzylsulfamoyl)-benzoesäure

HPLC1: Rt = 5.368 MS: 539.06

### Syntheseweg:

a) 4-Chloro-5-(2,3-Dichloro-benzylsulfamoyl)-benzoesäure aus Reaktion von 2,4-Dichloro-5-(chlorsulfonyl)-benzoesäure und 2,3-Dichloro-benzylamin nach allg. Vorschrift, Stufe 1.
b) 4-Chloro-2-phenylbutylamino-5-(2,3-dichloro-benzylsulfamoyl)-benzoesäure aus a) durch Reaktion mit Phenylbutylamin nach allg. Vorschrift, Stufe 2, farbloser Feststoff.

### Beispiel 13: 4-Chloro-2-phenylbutylamino-5-(2,4-dichloro-benzylsulfamoyl)-benzoesäure

HPLC1: Rt = 5.433 MS: 539.06

### Syntheseweg:

a) 4-Chloro-5-(2,4-Dichloro-benzylsulfamoyl)-benzoesäure aus Reaktion von 2,4-Dichloro-5-(chlorsulfonyl)-benzoesäure und 2,4-Dichloro-benzylamin nach allg. Vorschrift, Stufe 1.
b) 4-Chloro-2-phenylbutylamino-5-(2,4-dichloro-benzylsulfamoyl)-benzoesäure aus a) durch Reaktion mit Phenylbutylamin nach allg. Vorschrift, Stufe 2, farbloser Feststoff.

### Beispiel 14: 4-Chloro-2-phenylbutylamino-5-(2-chloro-6-fluor-benzylsulfamoyl)-benzoesäure

HPLC1: Rt = 5.230 MS: 523.11

### Syntheseweg:

a) 4-Chloro-5-(2-chloro-6-fluoro-benzylsulfamoyl)-benzoesäure aus Reaktion von 2,4-Dichloro-5-(chlorsulfonyl)-benzoesäure und 2-Chloro-6-fluoro-benzylamin nach allg. Vorschrift, Stufe 1.
b) 4-Chloro-2-phenylbutylamino-5-(2-chloro-6-fluoro-benzylsulfamoyl)-benzoesäure aus a) durch Reaktion mit Phenylbutylamin nach allg. Vorschrift, Stufe 2, farbloser Feststoff.

### Beispiel 15: 4-Chloro-5-[(5-methyl-furan-2-ylmethyl)-sulfamoyl]-2-phenylbutylaminobenzoesäure

HPLC1: Rt = 5.036 MS: 513.19

### Syntheseweg:

a) 2,4-Dichloro-5-[(5-methyl-furan-2-ylmethyl)-sulfamoyl]-benzoesäure aus Reaktion von 2,4-Dichloro-5-(chlorsulfonyl)-benzoesäure und 5-Methyl-furan-2-yl-methylamin nach allg. Vorschrift, Stufe 1.
b) 4-Chloro-5-[(5-methyl-furan-2-ylmethyl)-sulfamoyl]-2-phenylbutylaminobenzoesäure aus a) durch Reaktion mit Phenylbutylamin nach allg. Vorschrift, Stufe 2, farbloser Feststoff.

### Beispiel 16: 4-Chloro-5-[2-(4-chloro-phenyl)-ethylsulfamoyl]-2-phenylbutylaminobenzoesäure

HPLC1: Rt = 5.453 MS: 519.14

### Syntheseweg:

a) 2,4-Dichloro-5-[2-(4-chloro-phenyl)-ethylsulfamoyl]-benzoesäure aus Reaktion von 2,4-Dichloro-5-(chlorsulfonyl)-benzoesäure und 2-(4-Chloro-phenyl)-ethylamin nach allg. Vorschrift, Stufe 1.
b) 4-Chloro-5-[2-(4-chloro-phenyl)-ethylsulfamoyl]-2-phenylbutylamino-benzoesäure aus a) durch Reaktion mit Phenylbutylamin nach allg. Vorschrift, Stufe 2, farbloser Feststoff.

### Beispiel 17: 4-Chloro-2-phenylbutylamino-5-(2-thiophen-2-yl-ethylsulfamoyl)-benzoesäure

HPLC1: Rt = 5.253 MS: 491.10

### Syntheseweg:

a) 2,4-Dichloro-5-(2-thiophen-2-yl-ethylsulfamoyl)-benzoesäure aus Reaktion von 2,4-Dichloro-5-(chlorsulfonyl)-benzoesäure und 2-Thiophen-2-yl-ethylamin nach allg. Vorschrift, Stufe 1.
b) 4-Chloro-2-phenylbutylamino-5-(2-thiophen-2-yl-ethylsulfamoyl)-benzoesäure aus a) durch Reaktion mit Phenylbutylamin nach allg. Vorschrift, Stufe 2, farbloser Feststoff.

### Beispiel 18: 4-Chloro-5-(4-ethyl-phenylsulfamoyl)-2-phenylbutylamino-benzoesäure

HPLC1: Rt = 5.358 MS: 485.17

### Syntheseweg:

a) 2,4-Dichloro-5-(4-ethyl-phenylsulfamoyl)-benzoesäure aus Reaktion von 2,4-Dichloro-5-(chlorsulfonyl)-benzoesäure und 4-Ethyl-anilin nach allg. Vorschrift, Stufe 1.
b) 4-Chloro-5-(4-ethyl-phenylsulfamoyl)-2-phenylbutylamino-benzoesäure aus a) durch Reaktion mit Phenylbutylamin nach allg. Vorschrift, Stufe 2, farbloser Feststoff.

### Beispiel 19: 4-Chloro-5-(phenylbutylsulfamoyl)-2-phenylbutylamino-benzoesäure

HPLC1: Rt = 5.511 MS: 523.11
aus Reaktion von 2,4-Dichloro-5-(chlorsulfonyl)-benzoesäure mit Phenylbutylamin nach allg. Vorschrift, Stufe 2, farbloser Feststoff.

### Beispiel 20: 4-Chloro-5-(cyclohexylmethyl-sulfamoyl)-2-phenylbutylaminobenzoesäure

HPLC1: Rt = 5.548 MS: 475.10

### Syntheseweg:

a) 2,4-Dichloro-5-(cyclohexylmethyl-sulfamoyl)-benzoesäure aus Reaktion von 2,4-Dichloro-5-(chlorsulfonyl)-benzoesäure und Cyclohexylamin nach allg. Vorschrift, Stufe 1.
b) 4-Chloro-5-(cyclohexylmethyl-sulfamoyl)-2-phenylbutylamino-benzoesäure aus a) durch Reaktion mit Phenylbutylamin nach allg. Vorschrift, Stufe 2, farbloser Feststoff.

### Beispiel 21: 4-Chloro-5-(isobutyl-sulfamoyl)-2-phenylbutylamino-benzoesäure

HPLC1: Rt = 5.208 MS: 437.10

### Syntheseweg:

a) 2,4-Dichloro-5-(isobutyl-sulfamoyl)-benzoesäure aus Reaktion von 2,4-Dichloro-5-(chlorsulfonyl)-benzoesäure und Isobutylamin nach allg. Vorschrift, Stufe 1.
b) 4-Chloro-5-(isobutyl-sulfamoyl)-2-phenylbutylamino-benzoesäure aus a) durch Reaktion mit Phenylbutylamin nach allg. Vorschrift, Stufe 2, farbloser Feststoff.

### Pharmakologischer Teil:

Beschreibung der NBC-Aktivitätsmessungen:

Die meisten der molekularbiologischen Techniken folgen Protokollen aus den Werken "Current Protocols in Molecular Biology (eds. Ausubel, F.M., Brent, R., Kingston, R.E., Moore, D.D., Seidman, J.G., Smith, J.A. und Struhl, K.; John Wiley & Sons)" bzw: "Molecular Cloning: A Laboratory Manual (Sambrook, J., Fritsch, E.F. und Maniatis, T.; Cold Spring Harbor Laboratory Press (1989))". Zunächst wurde die Herzform des humanen NBC1 durch RT-PCR kloniert. Nach Bestätigung der Sequenz wurden die entsprechenden cDNAs in den Vektor pcDNA3.1 + einkloniert, der als Selektionsmarker für eukaryote Zellen das neo-Gen enthält. Für die Amplifikation der humanen Herzform des NBC1 wurde humane Herz-mRNA (Fa. Clontech, Palo Alto, CA, USA) mit Primem amptifiziert, die den Bereich abdecken, in dem sich die Herzform von der Nierenform unterscheidet. Die Herzform unterscheidet sich dabei nur am 5'-Ende der codierenden Sequenz. Der für die ersten 41 Aminosäuren der Nierenform codierende Bereich ist in der Herzform durch einen für 85 Aminosäuren codierenden Bereich ersetzt (Positionen 118 - 370 aus Abukadze et al., J. Biol. Chem. 273, 17689 -17695 (1998) bzw. Positionen 45 - 294 aus Choi et al., Am. J. Physiol. Cell Physiol. 276, C576-C584 (1999) ersetzen Positionen 150 - 270 aus Burnham et al. (s.o.), J. Biol. Chem. 272, 19 111 - 19 114 (1997) umfassen. Das Produkt der PCR-Reaktion wurde zunächst in den Vektor pCR2.1 kloniert und nach Verifizierung der Sequenz mittels durch die PCR-Reaktion eingeführter Schnittstellen in die cDNA der Nierenform des NBC1 einkloniert. Das erhaltene Konstrukt wurde durch Sequenzierung auf korrekten Einbau der humanen Herz NBC1-cDNA überprüft. Die erhaltenen Plasmide für die Herzform des humanen NBC1 wurden mittels des LipofectAmine™ Reagent der Firma LifeTechnologies (Gaithersburg, MD, USA) in die Zelllinie CHO K1 (Ovarzellen des Chinesischen Hamsters) transfiziert, die keine messbare NBC-Aktivität aufweist. Nach Selektion auf transfizierte Zellen über Wachstum in G418-haltigem Medium (nur Zellen, die durch Transfektion ein neo-Gen erhalten haben, können unter diesen Bedingungen überleben) wurden einzelne Zellen isoliert und kultiviert. Mit dem unten beschriebenen Test wurden am FLIPR Zellklone identifiziert, die eine deutliche NBC-Aktivität aufweisen. Die besten Zellinien wurden für die weiteren Tests verwendet und zur Vermeidung eines Verlustes der transfizierten Sequenz unter ständigem Selektionsdruck in G418-haltigem Medium kultiviert.

Zur Bestimmung der inhibitorischen Aktivität der Wirkstoffe auf die Herzform des humanen NBC1 wurde ein Test aufgebaut, der eine Weiterentwicklung des für das Testen von Inhibitoren des NCBE (Na⁺-abhängiger Cl⁻/HCO₃⁻-Austauscher) aufgebauten Assays (EP 0 903 339) auf Basis der "Acid Load" Methode (Sardet et al., Cell 56, 271 - 280 (1989); Faber et al., Cell. Physiol. Biochem. 6, 39 - 49 (1996) darstellt. In diesem Test wird die Erholung des intrazellulären pHs (pHᵢ ) nach einer vorhergehenden Ansäuerung unter Bedingungen ermittelt, bei denen der NBC aktiv ist, die anderen pHᵢ regulierenden Systeme der CHO-Zellen wie NHE (Na⁺/H⁺-Austauscher) und NCBE (Na⁺-abhängiger Cl⁻/HCO₃₋-Austauscher) durch spezifische Inhibitoren aber blockiert sind. Auf diese Weise wird sichergestellt, dass die beobachtete Erholung des intrazellulären pH auf der Aktivität des NBC1 beruht.

### Durchführung der Messungen

Am Vortag werden die transfizierten Zellen auf 96-Well Mikrotiterplatten in einer Dichte von ca. 15.000 Zellen/Well in 200 µl Wachstumsmedium ausgesät und über Nacht bei 37°C im CO₂-Brutschrank inkubiert. Der intrazelluläre pH der transfizierten Zellen mit dem pH-sensitiven Fluoreszenzfarbstoff BCECF (Molecular Probes (Eugene, OR, USA), eingesetzt wird die Vorstufe BCECF-AM) bestimmt. Die Zellen werden zunächst mit BCECF-AM beladen. Das Wachstumsmedium der am Vortag ausgesäten Zellen wird manuell abgenommen, da das im Medium vorhandene fötale Kälberserum die BCECF-Färbung stören könnte. Zur Färbung werden zu den Zellen eines jeden Wells 100 µl NH₄Cl-Färbepuffer (20 mM NH₄Cl, 115 mM NaCl, 1 mM MgSO₄, 1 mM CaCl₂, 5 mM KCl, 20 mMHEPES, 5 mM Glucose; pH mit 1 M NaOH auf 7,4 eingestellt) gegeben, der 5 µM BCECF-AM enthält. Die Zellen werden 20 Minuten bei 37°C im CO₂-Brutschrank mit dieser Färbelösung inkubiert. Während dieser Zeitspanne reichem sich zum einen NH₄⁺-lonen in den Zellen an, was eine leichte Alkalisierung hervorruft, zum anderen gelangt BCECF-AM in die Zellen, wo durch Wirkung von Esterasen der Farbstoff BCECF, der nicht zellmembranpermeabel ist, aus BCECF-AM freigesetzt wird. Zur Ansäuerung der Zellen werden diese anschließend im Zellwascher gründlich (dreimaliges Waschen mit einem Gesamtvolumen von 1,2 ml pro Well) mit einem Na⁺- und NH₄⁺-freien Waschpuffer gewaschen (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM HEPES, 5 mM Glucose; pH mit 1 M KOH auf 7,4 eingestellt). Dieser Waschritt führt zu einem drastischen Abfall des intrazellulären pHs (∼6,3-6,4). Da der Waschpuffer aber weder Natrium- noch Bicarbonationen enthält, sind die Zellen nicht in der Lage ihren intrazellulären pH zu regulieren. Die eigentliche Messung der intrazellulären pH-Erholung findet im sogenannten FLIPR (Fluorescence Imaging Plate Reader) der Firma Molecular Devices (Sunnyvale, CA, USA) statt. Der FLIPR besitzt einen Argonlaser, dessen 488 nm Bande sich sehr gut zur Anregung des BCECFs eignet. Durch eine komplizierte Strahlenführung wird erreicht, dass alle 96 Wells einer Mikrotiterplatte gleichzeitig angeregt und somit gleichzeitig gemessen werden können. Durch die besondere Konstruktionsweise des FLIPRs werden nur die unteren 50 µm in jedem Well angeregt, weshalb man bevorzugt mit adhärenten Zellen wie z.B. CHO arbeitet. Das von den angeregten Zellen emittierte Licht gelangt zunächst über einen Filter, der zwischen 510 und 570 nm durchlässig ist, und wird dann mittels einer CCD-Kamera registriert. Da der FLIPR auch einen eingebauten 96-Spitzen-Pipettor enthält, kann man in alle 96 Wells einer Mikrotiterplatte gleichzeitig das gleiche Volumen einer beliebigen Flüssigkeit pipettieren. Etwa jede Sekunde kann man eine Gesamtmessung einer vollständigen Mikrotiterplatte durchführen. Im FLIPR wird zu den nach dem Waschen angesäuerten Zellen jeweils 180 µl Substanzpuffer (90 mM NaCl, 25 mM NaHCO₃, 25 mM KCl, 1 mM MgCl₂, 1 mM CaCl₂, 0,8 mM K₂HPO₄, 0,2 mM KH₂PO₄, 10 mM HEPES, 5 mM Glucose; am Tag der Messung wird 5 Sekunden reines CO₂ durchgeleitet und der pH anschließend mit 1 M NaOH auf 7,4 eingestellt; das CO₂-Durchleiten kann aber auch entfallen, ohne dass sich die Messergebnisse merkbar ändern) hinzupipettiert. Um die unter diesen Pufferbedingungen ebenfalls aktiven pH-Regulationssysteme NHE und NCBE zu hemmen, enthält der Substanzpuffer zusätzlich noch spezifische Inhibitoren dieser Austauscher. Die Endkonzentration des NHE-Inhibitors Cariporide mesilat (EP 589 336) beträgt 10 µM, die des NBCE-Inhibitors nach EP 855 392 Beispiel 1: 2-Butyl-5-methylsulfanyl-3-(2'-cyanaminosulfonyl-biphenyl-4-ylmethyl)-3H-imidazol-4-carbonsäure-ethylester 30 µM. Nach Zugabe von Substanzpuffer steigt der intrazelluläre pH der zuvor angesäuerten Zellen durch die Aktivität des NBC an, was sich in einer Fluoreszenzzunahme des pH- sensitiven Farbstoffes bemerkbar macht.

Die inhibitorische Wirkung einer Substanz wird nun bestimmt, indem man den Fluoreszenzanstieg, der sich linear zum pH-Anstieg verhält, unter Einfluss dieser Substanz mit dem von Wells vergleicht, bei denen der NBC ungehemmt ist [100% Aktivität, nur Zugabe von Cariporide mesilat und des NCBE- Blockers nach EP 855 392. Beispiel 1] bzw. völlig gehemmt ist [0% Aktivität, neben Cariporide mesilat und NCBE-Blocker nach EP 855 392, Bsp. 1, noch Zugabe von 400 µM DCDPC, 4-Chloro-2-(3-chloro-phenylamino)-benzoesäure].

Die gesamte Messung einer Mikrotiterplatte dauert zwei Minuten, wobei die gesamte Platte alle 2 Sekunden gemessen wird. Nach den ersten 5 Messungen werden die jeweils 180 µl Substanzpuffer, die die zu testenden Verbindungen enthalten, mit einer Geschwindigkeit von 60 µl pro Sekunde zu den angesäuerten Zellen pipettiert. Nach wenigen Sekunden bereits zeigt sich in Wells, in denen der NBC nicht gehemmt wird, eine deutliche Fluoreszenzzunahme. Der Bereich zwischen 20 und 80 Sekunden, in dem die Fluoreszenzzunahme in den Positivkontrollen linear verläuft, wird zur Berechnung der verbleibenden NBC-Aktivität betrachtet. Von den 96 Wells der Mikrotiterplatte werden jeweils 8 für den 100%- bzw. den 0%-Wert verwendet.

Die folgenden Daten beziehen sich auf die Restaktivität bei einer Inhibitor-Konzentration von 10µM und sind Resultat von Doppelbestimmungen.

| Ergebnisse: | [%] at 10µM |
|---|---|
| Beispiel 1 | 46 |
| 2 | 56 |
| 3 | 88 |
| 4 | 54 |
| 5 | 78 |
| 6 | 69 |
| 7 | 95 |
| 8 | 89 |
| 9 | 89 |
| 10 | 75 |
| 11 | 81 |
| 12 | 37 |
| 13 | 60 |
| 14 | 87 |
| 15 | 79 |
| 16 | 91 |
| 17 | 95 |
| 18 | 79 |
| 19 | 77 |
| 20 | 89 |
| 21 | 94 |

## Patentansprüche

1. Anthranilsäuren der Formel I 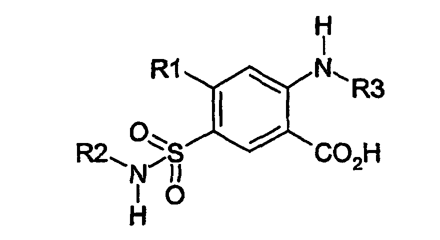 worin bedeuten:
R(1) H, Cl, Br, I, CN, (C₁-C₈)- Alkyl, (C₃-C₆)- Cycloalkyl oder Phenyl,
wobei der aromatische Kem unsubstituiert oder substituiert ist mit 1-3 Substituenten aus der Gruppe F, Cl, (C₁-C₃)- Alkyl, Methoxy oder -(CF₂)ₐ-CF₃;
a Null, 1, 2 oder 3;
R(2) (C₁-C₈)- Alkyl, -C_{b}H_{2b}- (C₃-C₆)- Cycloalkyl, -C_{b}H_{2b}- Phenyl, -C_{b}H_{2b}-Pyridinyl, -C_{b}H_{2b}- Thiophenyl, -C_{b}H_{2b}- Furanyl,
wobei die aromatischen Systeme unsubstituiert oder substituiert sind mit 1-3 Substituenten aus der Gruppe F, Cl, CF₃, (C₁-C₃)-Alkyl, Methoxy oder -SO₂NR(4)R(5);
R(4) und R(5) unabhängig voneinander H, (C₁-C₄)-Alkyl,
b Null, 1, 2, 3 oder 4;
R(3) -C_{d}H_{2d}- Phenyl,
wobei der aromatische Kem unsubstituiert oder substituiert ist mit 1-3 Substituenten aus der Gruppe F, Cl, CF₃, (C₁-C₃)- Alkyl oder Methoxy;
d 3 oder 4;
sowie deren pharmazeutisch verträgliche Salze.

2. Verbindungen der Formel I nach Anspruch 1, in denen bedeuten:
R(1) Cl, (C₁-C₄)- Alkyl oder Phenyl,
wobei der aromatische Kern unsubstituiert oder substituiert ist mit 1-3 Substituenten ausgewählt aus der Gruppe bestehend aus F, Cl, CF₃, (C₁-C₃)- Alkyl oder Methoxy;
R(2) (C₁-C₄)- Alkyl, -C_{b}H_{2b}- Cyclohexyl, -C_{b}H_{2b}- Phenyl, -C_{b}H_{2b}- Pyridinyl, -C_{b}H_{2b}- Thiophenyl, -C_{b}H_{2b}- Furanyl,
wobei die aromatischen Systeme unsubstituiert oder substituiert sind mit 1 - 3 Substituenten ausgewählt aus der Gruppe bestehend aus F, Cl, CF₃, (C₁-C₃)-Alkyl, Methoxy oder -SO₂NH₂;
b Null, 1 oder 2;
R(3) -n-C₄H₈- Phenyl,
wobei das Phenyl unsubstituiert ist oder substituiert mit 1 - 3 Substituenten ausgewählt aus der Gruppe bestehend aus F, Cl, CF₃, (C₁-C₃)- Alkyl oder Methoxy;
sowie deren pharmazeutische verträgliche Salze.

3. Verbindungen der Formel I nach Anspruch 1, **dadurch gekennzeichnet, dass** darin bedeuten:
R(1) Cl, (C₁-C₄)- Alkyl oder Phenyl;
wobei der aromatische Kern unsubstituiert oder substituiert ist mit 1-3 Substituenten ausgewählt aus der Gruppe bestehend aus F, Cl, CF₃, (C₁-C₃)- Alkyl oder Methoxy;
R(2) (C₁-C₄)- Alkyl, -C_{b}H_{2b}- Cyclohexyl, -C_{b}H_{2b}- Phenyl, -C_{b}H_{2b}- Pyridinyl, -C_{b}H_{2b}- Thiophenyl, -C_{b}H_{2b}- Furanyl;
wobei die aromatischen Systeme unsubstituiert oder substituiert sind mit 1 - 3 Substituenten ausgewählt aus der Gruppe bestehend aus F, Cl, CF₃, (C₁-C₃)- Alkyl, Methoxy oder -SO₂NH₂;
b 1;
R(3) -n-C₄H₈- Phenyl,
sowie deren pharmazeutisch verträgliche Salze.

4. Verbindung nach Anspruch 1, **dadurch gekennzeichnet, dass** sie 4- Chloro-5-(3-chloro-4-fluoro-benzylsulfamoyl)-2-phenylbutylamino-benzoesäure ist;
sowie ihre pharmazeutisch verträglichen Salze.

5. Verwendung einer Verbindung I nach Anspruch 1 zur Herstellung eines Medikaments zur Behandlung von Arrhythmien.

6. Verwendung einer Verbindung I nach Anspruch 1 zur Herstellung eines Medikaments zur Behandlung oder Prophylaxe des Herzinfarkts.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch I zur Herstellung eines Medikaments zur Behandlung von Krankheiten, bei denen die Zellproliferation eine primäre oder sekundäre Ursache darstellt.

15. Verwendung einer Verbindung I nach Anspruch 1 zur Herstellung eines Medikaments zur Behandlung oder Prophylaxe von Störungen des Fettstoffwechsels.

16. Heilmittel, enthaltend eine wirksame Menge einer Verbindung I nach einem oder mehreren der Ansprüche 1 bis 4.

## Claims

1. An anthranilic acid of the formula I 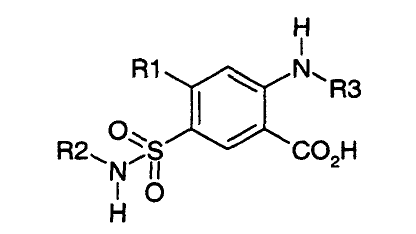 in which:
R(1) is H, Cl, Br, I, CN, (C₁-C₈)-alkyl, (C₃-C₆)-cycloalkyl or phenyl, where the aromatic nucleus is unsubstituted or substituted by 1-3 substituents from the group F, Cl, (C₁-C₃)-alkyl, methoxy or -(CF₂)ₐ-CF₃;
a is zero, 1, 2 or 3;
R(2) is (C₁-C₈)-alkyl, -C_{b}H_{2b}-(C₃-C₆)-cycloalkyl, -C_{b}H_{2b}-phenyl, -C_{b}H_{2b}-pyridinyl, -C_{b}H_{2b}-thiophenyl, -C_{b}H_{2b}-furanyl,
where the aromatic systems are unsubstituted or substituted by 1-3 substituents from the group F, Cl, CF₃, (C₁-C₃)-alkyl, methoxy or -SO₂NR(4)R(5);
R(4) and R(5) independently of one another are H, (C₁-C₄)-alkyl,
b is zero, 1, 2, 3 or 4;
R(3) is -C_{d}H_{2d}-phenyl,
where the aromatic nucleus is unsubstituted or substituted by 1-3 substituents from the group F, Cl, CF₃, (C₁-C₃)-alkyl or methoxy;
d is 3 or 4;
or its pharmaceutically tolerable salts.

2. A compound of the formula I as claimed in claim 1, in which:
R(1) is Cl, (C₁-C₄)-alkyl or phenyl,
where the aromatic nucleus is unsubstituted or substituted by 1-3 substituents selected from the group consisting of F, Cl, CF₃, (C₁-C₃)-alkyl or methoxy;
R(2) xis (C₁-C₄)-alkyl, -C_{b}H_{2b}-cyclohexyl, -C_{b}H_{2b}-phenyl, -C_{b}H_{2b}-pyridinyl, -C_{b}H_{2b}-thiophenyl, -C_{b}H_{2b}-furanyl,
where the aromatic systems are unsubstituted or substituted by 1-3 substituents selected from the group consisting of F, Cl, CF₃, (C₁-C₃)-alkyl, methoxy or -SO₂NH₂;
b is zero, 1 or 2;
R(3) is -n-C₄H₈-phenyl,
where the phenyl is unsubstituted or is substituted by 1-3 substituents selected from the group consisting of F, Cl, CF₃, (C₁-C₃)-alkyl or methoxy;
or its pharmaceutically tolerable salts.

3. A compound of the formula I as claimed in claim 1, wherein:
R(1) is Cl, (C₁-C₄)-alkyl or phenyl;
where the aromatic nucleus is unsubstituted or substituted by 1-3 substituents selected from the group consisting of F, Cl, CF₃, (C₁-C₃)-alkyl or methoxy;
R(2) (C₁-C₄)-alkyl, -C_{b}H_{2b}-cyclohexyl, -C_{b}H_{2b}-phenyl, -C_{b}H_{2b}-pyridinyl, -C_{b}H_{2b}-thiophenyl, -C_{b}H_{2b}-furanyl;
where the aromatic systems are unsubstituted or substituted by 1-3 substituents selected from the group consisting of F, Cl, CF₃, (C₁-C₃)-alkyl, methoxy or -SO₂NH₂;
b is 1;
R(3) is -n-C₄H₈-phenyl,
and its pharmaceutically tolerable salts.

4. The compound as claimed in claim 1, which is 4-chloro-5-(3-chloro-4-fluorobenzylsulfamoyl)-2-phenylbutylaminobenzoic acid; or its pharmaceutically tolerable salts.

5. The use of a compound I as claimed in claim 1 for the production of a medicament for the treatment of arrhythmias.

6. The use of a compound I as claimed in claim 1 for the production of a medicament for the treatment or prophylaxis of cardiac infarct.

7. The use of a compound I as claimed in claim 1 for the production of a medicament for the treatment or prophylaxis of angina pectoris.

8. The use of a compound I as claimed in claim 1 for the production of a medicament for the treatment or prophylaxis of ischemic conditions of the heart.

9. The use of a compound I as claimed in claim 1 for the production of a medicament for the treatment or prophylaxis of ischemic conditions of the peripheral and central nervous system and of stroke.

10. The use of a compound I as claimed in claim 1 for the production of a medicament for the treatment or prophylaxis of ischemic conditions of peripheral organs and limbs.

11. The use of a compound I as claimed in claim 1 for the production of a medicament for the treatment of states of shock.

12. The use of a compound I as claimed in claim 1 for the production of a medicament for use in surgical operations and organ transplantation.

13. The use of a compound I as claimed in claim 1 for the production of a medicament for the preservation and storage of transplants for surgical measures.

14. The use of a compound I as claimed in claim I for the production of a medicament for the treatment of illnesses, in which cell proliferation is a primary or secondary cause.

15. The use of a compound I as claimed in claim 1 for the production of a medicament for the treatment or prophylaxis of disorders of lipid metabolism.

16. A therapeutic, comprising an efficacious amount of a compound I as claimed in one or more of claims 1 to 4.

## Revendications

1. Acides anthraniliques de formule I 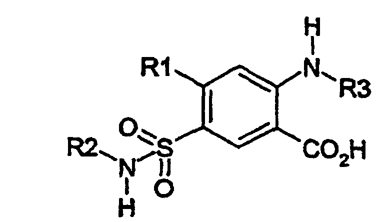 dans laquelle
R(1) représente H, Cl, Br, I, CN, un groupe alkyle en C₁-C₈, cycloalkyle en C₃-C₆ ou phényle,
le noyau aromatique n'étant pas substitué ou portant 1-3 substituants choisis dans l'ensemble constitué par F, Cl, les groupes alkyle en C₁-C₃, méthoxy et -(CF₂)ₐ-CF₃ ;
a est zéro, 1, 2 ou 3 ;
R(2) représente un groupe alkyle en C₁-C₈, -C_{b}H_{2b}-cycloalkyle(C₃-C₆), -C_{b}H_{2b}-phényle, -C_{b}H_{2b}-pyridinyle, -C_{b}H_{2b}-thiophényle, -C_{b}H_{2b}-furannyle,
les systèmes aromatiques n'étant pas substitués ou portant 1-3 substituants choisis dans l'ensemble constitué par F, Cl, CF₃, les groupes alkyle en C₁-C₃, méthoxy et -SO₂NR(4)R(5) ;
R(4) et R(5) représentant, indépendamment l'un de l'autre, H ou un groupe alkyle en C₁-C₄ ;
b est zéro, 1, 2, 3 ou 4 ;
R(3) représente un groupe -C_{d}H_{2d}-phényle,
le noyau aromatique n'étant pas substitué ou portant 1-3 substituants choisis dans l'ensemble constitué par F, Cl, CF₃, les groupes alkyle en C₁-C₃ et méthoxy ;
d est 3 ou 4 ;
ainsi que leurs sels pharmaceutiquement acceptables.

2. Composés de formule I selon la revendication 1, dans lesquels :
R(1) représente Cl, un groupe alkyle en C₁-C₄ ou phényle,
le noyau aromatique n'étant pas substitué ou portant 1-3 substituants choisis dans l'ensemble constitué par F, Cl, CF₃, les groupes alkyle en C₁-C₃ et méthoxy ;
R(2) représente un groupe alkyle en C₁-C₄, -C_{b}H_{2b}-cyclohexyle, -C_{b}H_{2b}-phényle, -C_{b}H_{2b}-pyridinyle, -C_{b}H_{2b}-thiophényle, -C_{b}H_{2b}-furannyle,
les systèmes aromatiques n'étant pas substitués ou portant 1-3 substituants choisis dans l'ensemble constitué par F, Cl, CF₃, les groupes alkyle en C₁-C₃, méthoxy et -SO₂NH₂ ;
b est zéro, 1 ou 2 ;
R(3) représente un groupe -n-C₄H₈-phényle,
le fragment phényle n'étant pas substitué ou portant 1-3 substituants choisis dans l'ensemble constitué par F, Cl, CF₃, les groupes alkyle en C₁-C₃ et méthoxy ;
ainsi que leurs sels pharmaceutiquement acceptables.

3. Composés de formule I selon la revendication 1, **caractérisés en ce que** dans ceux-ci :
R(1) représente Cl, un groupe alkyle en C₁-C₄ ou phényle,
le noyau aromatique n'étant pas substitué ou portant 1-3 substituants choisis dans l'ensemble constitué par F, Cl, CF₃, les groupes alkyle en C₁-C₃ et méthoxy ;
R(2) représente un groupe alkyle en C₁-C₄, -C_{b}H_{2b}-cyclohexyle, -C_{b}H_{2b}-phényle, -C_{b}H_{2b}-pyridinyle, -C_{b}H_{2b}-thiophényle, -C_{b}H_{2b}-furannyle,
les systèmes aromatiques n'étant pas substitués ou portant 1-3 substituants choisis dans l'ensemble constitué par F, Cl, CF₃, les groupes alkyle en C₁-C₃, méthoxy et -SO₂NH₂ ;
b est 1 ;
R(3) représente le groupe -n-C₄H₈-phényle,
ainsi que leurs sels pharmaceutiquement acceptables.

4. Composé selon la revendication 1, **caractérisé en ce qu'**il est l'acide 4-chloro-5-(3-chloro-4-fluoro-benzylsulfamoyl)-2-phénylbutylaminobenzoïque,
ainsi que ses sels pharmaceutiquement acceptables.

5. Utilisation d'un composé I selon la revendication 1, pour la fabrication d'un médicament destiné au traitement ou à la prophylaxie d'arythmies.

6. Utilisation d'un composé I selon la revendication 1, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux central et périphérique et de l'accident vasculaire cérébral.

10. Utilisation d'un composé I selon la revendication 1, pour la fabrication d'un médicament destiné au traitement ou à la prophylaxie d'états ischémiques des organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des transplantations d'organes.

13. Utilisation d'un composé I selon la revendication 1, pour la fabrication d'un médicament destiné à la conservation et au stockage de transplants pour des interventions chirurgicales.

14. Utilisation d'un composé I selon la revendication 1, pour la fabrication d'un médicament destiné au traitement ou à la prophylaxie de maladies dans lesquelles la prolifération cellulaire représente une cause primaire ou secondaire.

15. Utilisation d'un composé I selon la revendication 1, pour la fabrication d'un médicament destiné au traitement ou à la prophylaxie de troubles du métabolisme lipidique.

16. Médicament, contenant une quantité efficace d'un composé I selon une ou plusieurs des revendications 1 à 4.
